# EUROPEAN PATENT APPLICATION

(11) **EP 4 438 604 A1**
(43) Date of publication of application: **02.10.2024**
(21) Application number: 22897801.1
(22) Date of filing: 22.11.2022
(51) Int. Cl.: C07D 471/14, C07D 519/00, A61K 31/4545, A61K 31/438, A61K 31/437, A61P 37/02, A61P 17/04, A61P 17/06, A61P 17/00

(54) **NEW CRYSTAL FORM OF COMPOUND, AND PREPARATION METHOD THEREFOR AND USE THEREOF**

(30) Priority: 25.11.2021 WO PCT/CN2021/133228
(71) Applicant: E-nitiate Biopharmaceuticals (Hangzhou) Co., Ltd, Hangzhou, Zhejiang 311100 (CN)
(72) Inventor: LIU, Pengfei, Hangzhou, Zhejiang 311100 (CN); SHEN, Wang, Hangzhou, Zhejiang 311100 (CN); GAO, Hongjun, Hangzhou, Zhejiang 311100 (CN); DING, Shizhe, Hangzhou, Zhejiang 311100 (CN)
(74) Representative: INNOV-GROUP
(86) International application number: PCT/CN2022/133494
(87) International publication number: WO 2023/093718

(57) **Abstract**

Provided are a crystal form of a compound of formula (I), a method for preparing the crystal form, a pharmaceutical composition containing the crystal form, and the use thereof in the preparation of a drug for treating diseases, disorders or symptoms, or a method thereof for treating diseases, disorders or symptoms.

## Description

### FIELD OF THE INVENTION

The present invention relates to the technical field of chemical medicine, and in particular to a new crystalline form of a compound, a preparation method of the crystal form, a pharmaceutical composition containing the crystal form, and the use of the crystalline form and the pharmaceutical composition.

### BACKGROUND OF THE INVENTION

Polymorphism or polymorphism is a unique property of certain molecules and molecular compositions. The same molecules may form different crystals due to different arrangements, and these crystals have different crystal structures and physical properties, such as solubility and stability, hygroscopicity, X-ray diffraction pattern. The phenomenon of polymorphism of drug molecules has attracted increasing attention. Many pharmaceutically active organic compounds can crystallize in more than one three-dimensional crystal structure. However, it is often impossible to predict whether a particular organic pharmaceutical compound will form different crystalline forms, much less the structure and properties of the crystalline forms themselves. Exploring new crystalline forms or polymorphs of pharmaceutically acceptable compounds can provide opportunities to improve the overall performance of pharmaceutical products.

For compound (R)-2-(1-(2-(1-hydroxyethyl)imidazo[4,5-d]pyrrolo[2,3-b]pyridin-1(6H)-yl)piperidine -4-yl) acetonitrile, the structure is shown in formula (I):

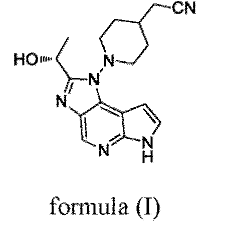

At present, the research on the crystalline form of this compound is still in a blank state. The new crystalline form of the compound of formula (I) provided by the present invention has the characteristics of good stability, high solubility and high bioavailability, and has broad application prospects.

### SUMMARY OF INVENTION

The purpose of the present invention is to provide a new crystalline form of compound (R)-2-(1-(2-(1-hydroxyethyl)imidazo[4,5-d]pyrrolo[2,3-b]pyridine-1(6H)-yl)piperidin-4-yl)ac etonitrile (hereinafter referred to as the compound of formula (I)).

The invention provides a crystalline form A of the compound of formula (I),

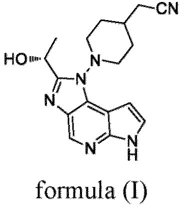

The crystalline form A is characterized by having an X-ray powder diffraction pattern comprising characteristic peak at 2θ angle of 8.6°±0.2°, 9.8°±0.2°, 10.7°±0.2°, 11.2°±0.2°, 12.3°±0.2°, 12.9°±0.2, 16.0°±0.2° and 19.0°±0.2°.

In some embodiments, the crystalline form A is characterized by having an X-ray powder diffraction pattern comprising characteristic peak at 2θ angle of 8.6°±0.2°, 9.8°±0.2°, 10.7°±0.2°, 11.2°±0.2°, 12.3°±0.2 12.9°±0.2°, 16.0°±0.2°, 17.0°±0.2°, 17.4°±0.2°, 17.8°±0.2°, 19.0°±0.2°, 19.9°±0.2° and 21.2°±0.2°.

In some embodiments, the main data of the X-ray powder diffraction pattern of crystalline form A is shown in Table 1.

**Table 1**

| No. | Diffraction angle 2θ (°) | Interplanar spacing (Å) | Relative intensity (%) |
|---|---|---|---|
| 1 | 8.6 | 10.28 | 15.8% |
| 2 | 9.8 | 8.98 | 100.0% |
| 3 | 10.7 | 8.23 | 11.6% |
| 4 | 11.2 | 7.86 | 28.1% |
| 5 | 12.3 | 7.19 | 21.3% |
| 6 | 12.9 | 6.85 | 7.5% |
| 7 | 13.7 | 6.44 | 7.3% |
| 8 | 13.9 | 6.35 | 10.0% |
| 9 | 16.0 | 5.52 | 6.4% |
| 10 | 17.0 | 5.21 | 8.8% |
| 11 | 17.4 | 5.09 | 27.4% |
| 12 | 17.8 | 4.98 | 51.4% |
| 13 | 18.4 | 4.82 | 2.2% |
| 14 | 18.6 | 4.77 | 2.5% |
| 15 | 19.0 | 4.68 | 31.1% |
| 16 | 19.9 | 4.46 | 7.3% |
| 17 | 20.4 | 4.34 | 22.8% |
| 18 | 20.6 | 4.30 | 26.0% |
| 19 | 21.2 | 4.20 | 11.3% |
| 20 | 22.8 | 3.90 | 6.0% |
| 21 | 23.4 | 3.81 | 4.7% |
| 22 | 24.7 | 3.60 | 2.5% |
| 23 | 25.0 | 3.55 | 2.8% |
| 24 | 26.3 | 3.39 | 2.7% |
| 25 | 26.6 | 3.35 | 3.7% |
| 26 | 27.0 | 3.31 | 4.9% |
| 27 | 27.6 | 3.23 | 4.4% |
| 28 | 27.8 | 3.20 | 5.1% |
| 29 | 28.3 | 3.15 | 3.6% |
| 30 | 28.6 | 3.12 | 3.1% |
| 31 | 29.0 | 3.07 | 1.5% |
| 32 | 30.2 | 2.96 | 1.8% |
| 33 | 31.2 | 2.87 | 5.3% |
| 34 | 31.5 | 2.84 | 1.7% |
| 35 | 31.9 | 2.80 | 2.1% |
| 36 | 32.7 | 2.74 | 2.2% |
| 37 | 36.3 | 2.47 | 1.3% |
| 38 | 37.4 | 2.40 | 1.2% |
| 39 | 37.8 | 2.38 | 2.5% |
| 40 | 38.1 | 2.36 | 5.3% |
| 41 | 38.7 | 2.33 | 1.0% |

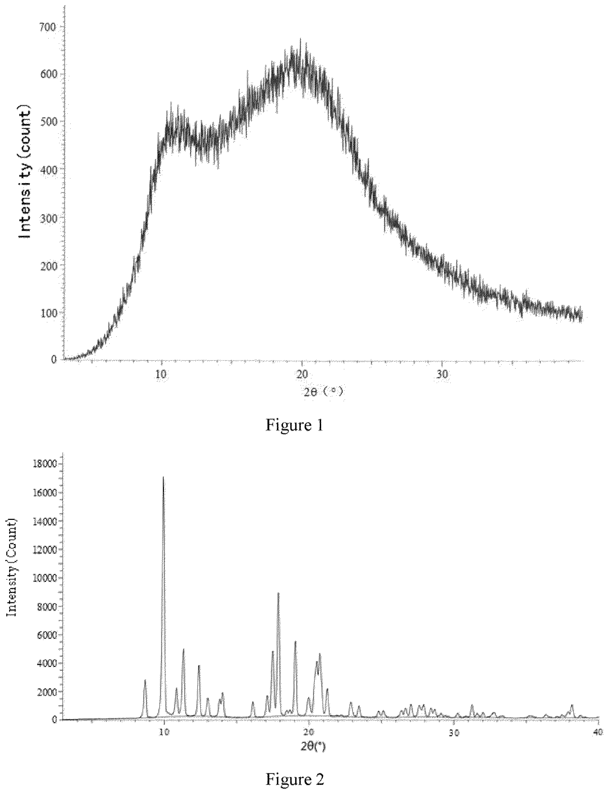

In some embodiments, the crystalline form A has an X-ray powder diffraction pattern substantially as shown in Figure 2.

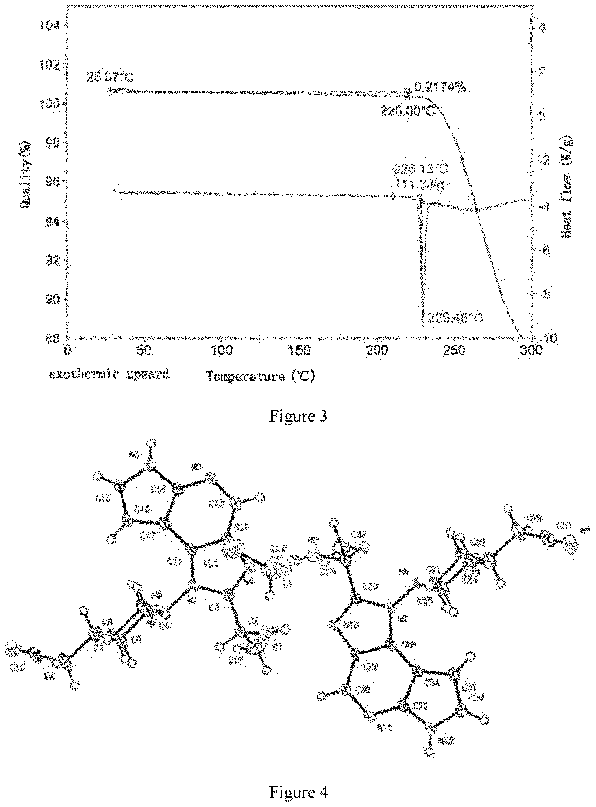

In some embodiments, the crystalline form A has an overlay of a differential scanning calorimetry (DSC) spectrum and a thermogravimetric analysis (TGA) spectrum substantially as shown in Figure 3.

In some embodiments, the stereoscopic structural diagram of the crystalline form A is shown in Figure 4.

In some embodiments, the crystalline form A is an amorphous form.

The present invention further provides a method for preparing crystalline form A, which includes the following steps:
1) Dissolve the compound of formula (I) in the crystallization solvent;
2) Concentration and filtration;

Wherein, the crystallization solvent is selected from ethanol (EtOH), n-heptane (n-heptane), isopropyl alcohol (IPA), 2-butanol (2-Butanol), butanone (MEK), ethyl acetate (EtOAc), 2-methyltetrahydrofuran (2-MeTHF), tetrahydrofuran (THF), methyl tert-butyl ether (MTBE) or a mixed solvent of any two or more solvents.

In some embodiments of the present invention, the crystallization solvent is selected from ethanol.

The present invention further provides a method for preparing crystalline form A, which includes: dissolving the compound of formula (I) in ethanol until it is clear, and then concentrating; stirring the obtained concentrated liquid to precipitate solid; filtering and drying to obtain the crystal Type A.

In some embodiments, the concentration is at a temperature of 35°C to 55°C.

In some embodiments, the concentration of the concentrate is 0.5-2g/mL.

In some embodiments, the stirring temperature is 5°C to 15°C.

The present invention further provides a crystalline form H of the compound of formula (I), the crystalline form H is characterized by having an X-ray powder diffraction pattern comprising characteristic peak at 2θ angle of 9.4°±0.2°, 11.0°±0.2°, 14.1°±0.2°, 16.0°±0.2°, 17.8°±0.2°, 19.3°±0.2 and 21.9°±0.2°.

In some embodiments, the crystalline form H is characterized by having an X-ray powder diffraction pattern comprising characteristic peak at 2θ angle of 9.4°±0.2°, 11.0°±0.2°, 11.7°±0.2°, 14.1°±0.2°, 15.4°±0.2, 16.0°±0.2°, 17.8°±0.2°, 19.3°±9.2°, 20.9°±0.2°, and 21.9°±0.2°.

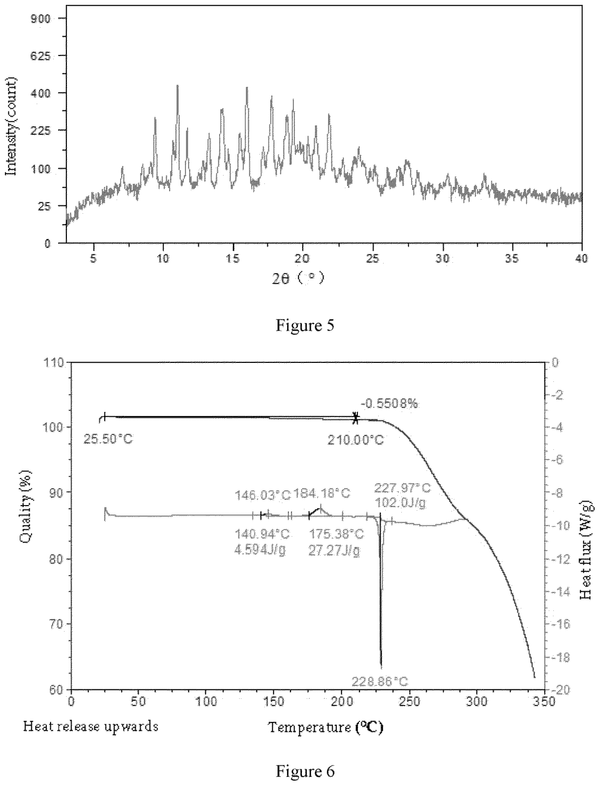

In some embodiments, the crystalline form H has an X-ray powder diffraction pattern substantially as shown in Figure 5.

In some embodiments, the crystalline form H has an overlay of a differential scanning calorimetry (DSC) spectrum and a thermogravimetric analysis (TGA) spectrum substantially as shown in Figure 6.

In some embodiments, the crystalline form H is an anhydrous form.

The present invention further provides crystalline form S of the compound of formula (I), the crystalline form S is characterized by having an X-ray powder diffraction pattern comprising characteristic peak at 2θ angle of 9.1°±0.2°, 10.1°±0.2°, 13.4°±0.2°, 15.5°±0.2°, 16.0°±0.2°, 17.8°±0.2°, 19.7°±0.2°, and 21.8°±0.2°.

In some embodiments, the crystalline form S is characterized by having an X-ray powder diffraction pattern comprising characteristic peak at 2θ angle of 9.1°±0.2°, 10.1°±0.2°, 13.4°±0.2°, 15.5°±0.2°, 16.0°±0.2, 17.8°±0.2°, 19.7°±0.2°, 21.0°±0.2°, 21.8°±0.2°, 23.3°±0.2°, 24.0°±0.2°, and 24.8°±0.2°.

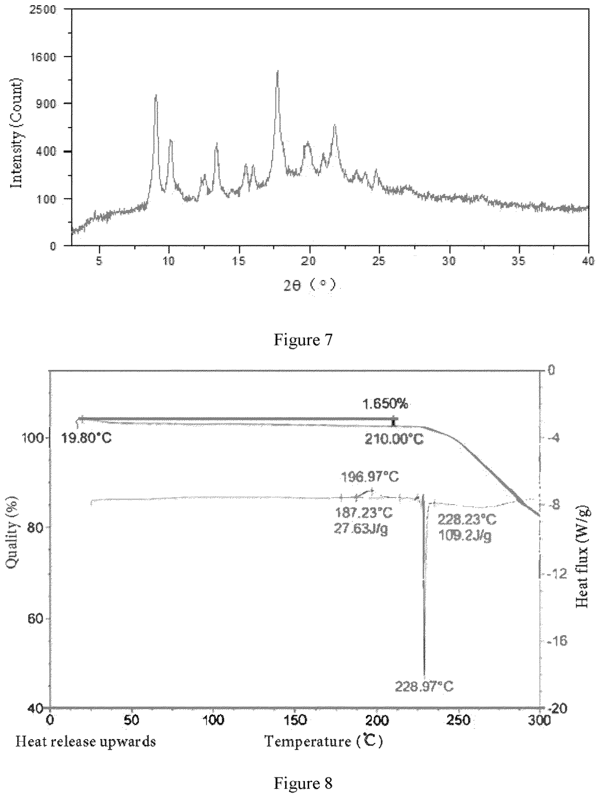

In some embodiments, the crystalline form S has an X-ray powder diffraction pattern substantially as shown in Figure 7.

In some embodiments, the crystalline form S has an overlay of a differential scanning calorimetry (DSC) spectrum and a thermogravimetric analysis (TGA) spectrum substantially as shown in Figure 8.

In some embodiments, the crystalline form S is an amorphous form.

The present invention further provides a crystalline form O of the compound of formula (I), the crystalline form O is characterized by having an X-ray powder diffraction pattern comprising characteristic peak at 2θ angle of 10.3°±0.2°, 11.9°±0.2°, 14.2°±0.2°, 15.3°±0.2°, 19.6°±0.2°, 20.3°±0.2°, 20.5°±0.2°, and 21.4°±0.2°.

In some embodiments, the crystalline form O is characterized by having an X-ray powder diffraction pattern comprising characteristic peak at 2θ angle of 10.3°±0.2°, 11.9°±0.2°, 14.2°±0.2°, 15.3°±0.2°, 19.6°±0.2, 20.3°±0.2°, 20.5°±0.2°, 21.4°±0.2°, 25.6°±0.2°, 28.6°±0.2°, 29.4°±0.2°, and 30.0°±0.2°.

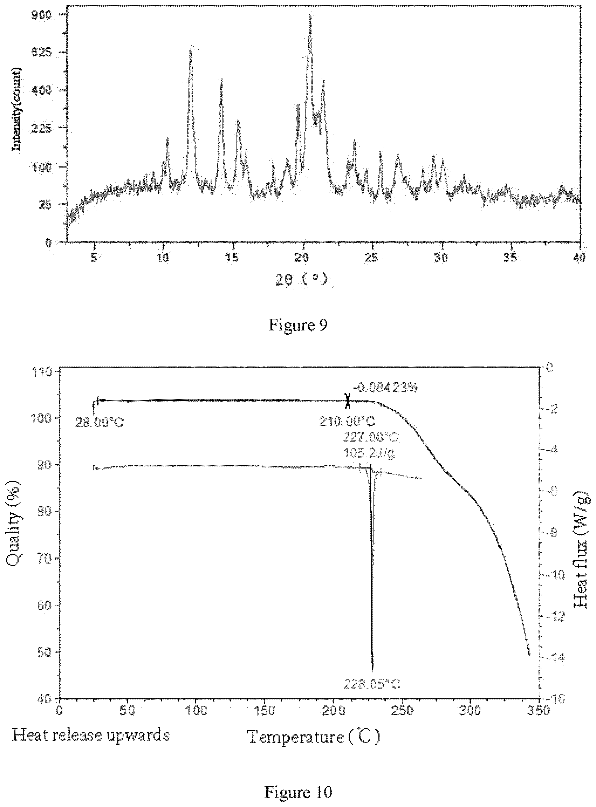

In some embodiments, the crystalline form O has an X-ray powder diffraction pattern substantially as shown in Figure 9.

In some embodiments, the crystalline form O has an overlay of a differential scanning calorimetry (DSC) spectrum and a thermogravimetric analysis (TGA) spectrum substantially as shown in Figure 10.

In some embodiments, the crystalline form O is an anhydrous form.

The present invention further provides a crystalline form P of the compound of formula (I), the crystalline form P is characterized by having an X-ray powder diffraction pattern comprising characteristic peak at 2θ angle of 9.5°±0.2°, 16.5°±0.2°, 19.6°±0.2°, 20.3°±0.2°, 23.9°±0.2°, and 25.1°±0.2°.

In some embodiments, the crystalline form P is characterized by having an X-ray powder diffraction pattern comprising characteristic peak at 2θ angle of 9.5°±0.2°, 16.5°±0.2°, 19.6°±0.2°, 20.3°±0.2°, 23.9°±0.2, 25.1°±0.2°, 26.3°±0.2°, and 28.9°±0.2°.

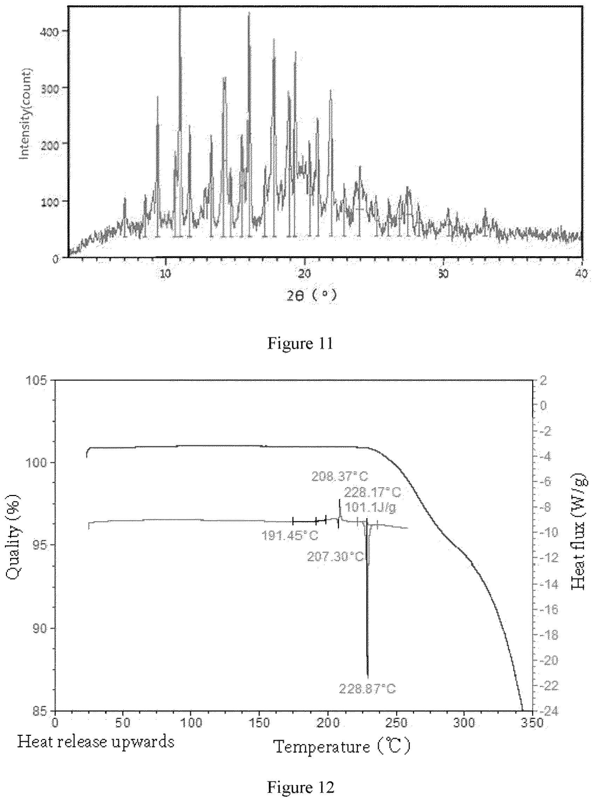

In some embodiments, the crystalline form P has an X-ray powder diffraction pattern substantially as shown in Figure 11.

In some embodiments, the crystalline form P has an overlay of a differential scanning calorimetry (DSC) spectrum and a thermogravimetric analysis (TGA) spectrum substantially as shown in Figure 12.

In some embodiments, the crystalline form P is an amorphous form.

The present invention further provides a crystalline form Q of the compound of formula (I), the crystalline form Q is characterized by having an X-ray powder diffraction pattern comprising characteristic peak at 2θ angle of 8.5°±0.2°, 14.5°±0.2°, 15.0°±0.2°, 16.8°±0.2°, 19.4°±0.2°, 23.6°±9.2°, 24.8°±0.2°, 26.3°±9.2°, and 27.4°±0.2°.

In some embodiments, the crystalline form Q is characterized by having an X-ray powder diffraction pattern comprising characteristic peak at 2θ angle of 8.5°±0.2°, 14.5°±0.2°, 15.0°±0.2°, 15.8°±0.2°, 16.8°±0.2, 18.8°±0.2°, 19.4°±0.2°, 20.1°±0.2°, 23.6°±0.2°, 24.8°±0.2°, 26.3°±9.2°, and 27.4°±0.2°.

In some embodiments, the main data of the X-ray powder diffraction pattern of crystalline form Q is shown in Table 2.

**Table 2**

| No. | Diffraction angle 2θ (°) | Interplanar spacing (Å) | Relative intensity (%) |
|---|---|---|---|
| 1 | 8.5 | 10.37 | 100 |
| 2 | 14.5 | 6.13 | 39.46 |
| 3 | 15.0 | 5.91 | 48.64 |
| 4 | 15.8 | 5.60 | 12.22 |
| 5 | 16.8 | 5.26 | 17.81 |
| 6 | 17.2 | 5.16 | 5.43 |
| 7 | 17.5 | 5.07 | 4.04 |
| 8 | 18.8 | 4.71 | 9.87 |
| 9 | 19.4 | 4.57 | 44 |
| 10 | 20.1 | 4.42 | 12.97 |
| 11 | 21.3 | 4.18 | 2.44 |
| 12 | 22.6 | 3.94 | 5.51 |
| 13 | 23.2 | 3.84 | 2.04 |
| 14 | 23.6 | 3.78 | 18.48 |
| 15 | 24.8 | 3.59 | 80.59 |
| 16 | 26.3 | 3.39 | 37.28 |
| 17 | 27.4 | 3.26 | 16.8 |
| 18 | 28.8 | 3.10 | 7.1 |
| 19 | 30.4 | 2.94 | 10.21 |
| 20 | 33.5 | 2.67 | 4.11 |
| 21 | 38.0 | 2.37 | 3.57 |

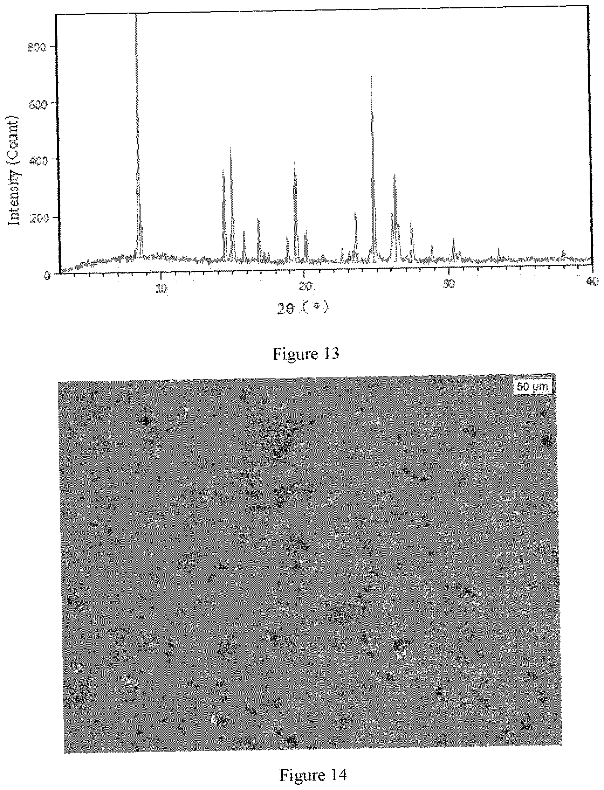

In some embodiments, the crystalline form Q has an X-ray powder diffraction pattern substantially as shown in Figure 13.

In some embodiments, the crystalline form Q has a polarization micrograph substantially as shown in Figure 14.

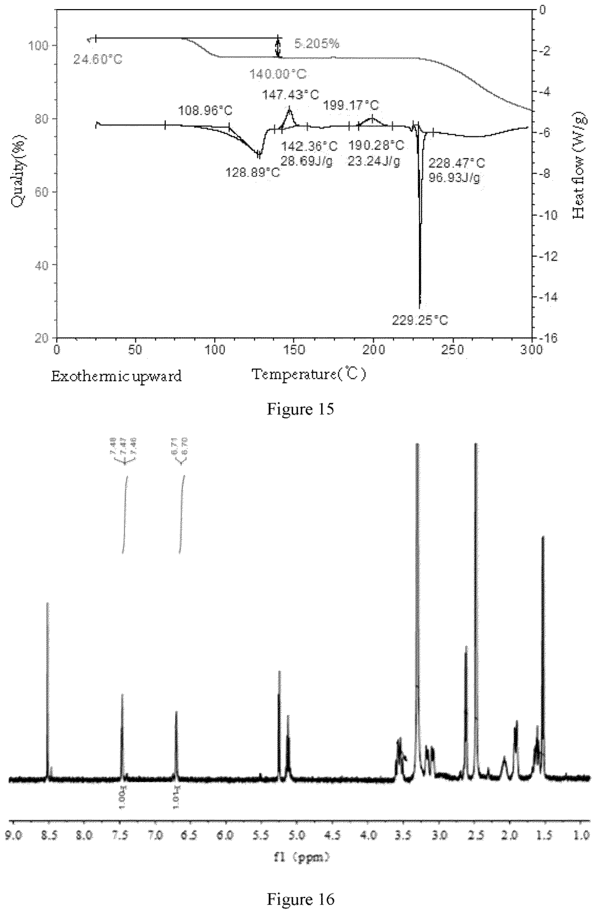

In some embodiments, the crystalline form Q has an overlay of a differential scanning calorimetry (DSC) spectrum and a thermogravimetric analysis (TGA) spectrum substantially as shown in Figure 15.

In some embodiments, the crystalline form Q has a proton nuclear magnetic resonance spectrum (1H-NMR spectrum) substantially as shown in Figure 16.

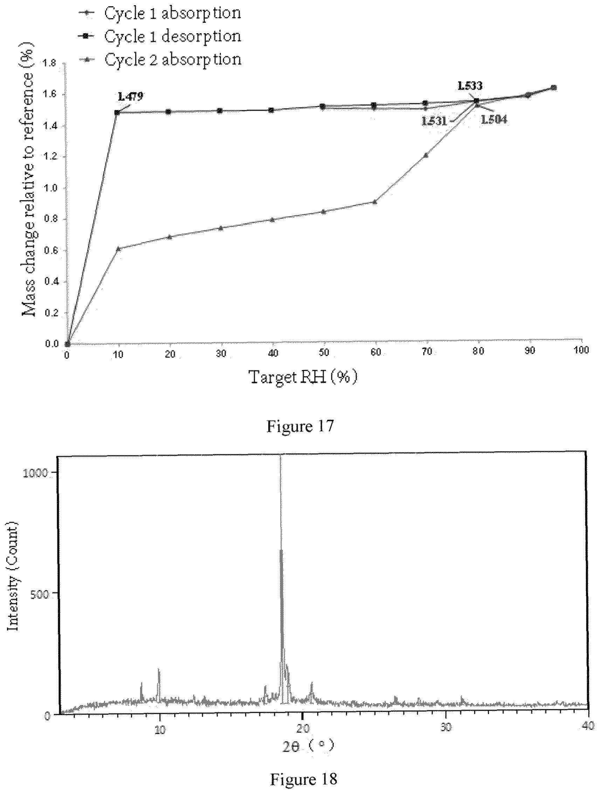

In some embodiments, the crystalline form Q has a dynamic gas phase adsorption pattern substantially as shown in Figure 17.

In some embodiments, the crystalline form Q is the hydrate crystalline form of the compound of formula (I).

The present invention further provides a method for preparing crystalline form Q, which includes: stirring crystal form A in an aqueous ethanol solution; centrifuging and drying to obtain the crystalline form Q.

In some embodiments, the water activity of the ethanol aqueous solution is 0.6-0.8.

The present invention further provides a crystalline form M of the compound of formula (I), the crystalline form M is characterized by having an X-ray powder diffraction pattern comprising characteristic peak at 2θ angle of 8.7°±0.2°, 9.9°±0.2°, 17.4°±0.2°, 18.6°±0.2°, 18.9°±0.2°, 20.6°±0.2°, and 26.5°±0.2°.

In some embodiments, the crystalline form M is characterized by having an X-ray powder diffraction pattern comprising characteristic peak at 2θ angle of 8.7°±0.2°, 9.9°±0.2°, 13.1°±0.2°, 17.4°±0.2°, 18.6°±0.2, 18.9°±0.2°, 20.6°±0.2°, 26.5°±0.2°, 28.1°±0.2°, and 31.1°±0.2°.

In some embodiments, the crystalline form M has an X-ray powder diffraction pattern substantially as shown in Figure 18.

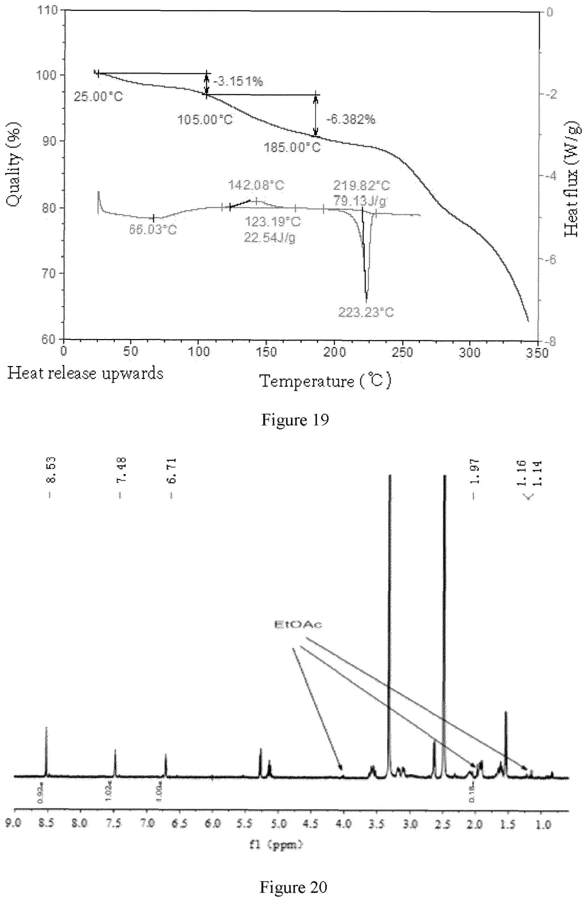

In some embodiments, the crystalline form M has an overlay of a differential scanning calorimetry (DSC) spectrum and a thermogravimetric analysis (TGA) spectrum substantially as shown in Figure 19.

In some embodiments, the crystalline form M has a proton nuclear magnetic resonance spectrum (1H-NMR spectrum) substantially as shown in Figure 20.

In some embodiments, the crystalline form M is a hydrate crystalline form of the compound of formula (I).

The present invention further provides crystalline compositions of the crystalline forms. In some embodiments of the present invention, the crystalline form A accounts for more than 50% by weight of the crystalline composition, preferably more than 80%, more preferably more than 90%, most preferably more than 95%.

The present invention further provides a pharmaceutical composition, which comprises a therapeutically effective amount of crystalline form A or crystalline form Q or a mixture thereof and a pharmaceutically acceptable carrier, diluent or excipient.

The present invention further provides a veterinary pharmaceutical composition, comprising an effective therapeutic amount of crystalline form A or crystalline form Q or a mixture thereof and a pharmaceutically acceptable carrier, diluent or excipient.

The present invention further provides the use of crystalline form A or crystalline form Q or a mixture thereof in the preparation of a medicament.

In some embodiments, the medicament is used to treat, prevent, or delay allergic reactions, allergic dermatitis, atopic dermatitis, eczema, pruritus, or psoriasis.

In some embodiments, the medicament is used to treat, prevent, or delay allergic reactions, allergic dermatitis, atopic dermatitis, eczema, pruritus, or psoriasis in mammals.

In some embodiments, the mammal includes a companion animal.

In some embodiments, the mammal includes livestock.

In some embodiments, the medicaments are used to treat diseases mediated by JAK kinase (JAK1).

The present invention further provides a method for treating and/or preventing disease by administering to a mammal in need thereof a therapeutically effective amount of crystalline form A or crystalline form Q, or mixtures thereof.

In some embodiments, the therapeutically effective amount is from 0.01 mg/kg body weight/day to 100 mg/kg body weight/day.

In some embodiments, the therapeutically effective amount is from 0.1 mg/kg body weight/day to 30 mg/kg body weight/day.

In some embodiments, the disease is mediated by JAK kinase (JAK1).

In some embodiments, the mammal includes a companion animal.

In some embodiments, the mammal includes livestock.

In some embodiments, crystalline form A or crystalline form Q, or mixtures thereof, is administered orally, parenterally, or topically.

The new crystal form of (R)-2-(1-(2-(1-hydroxyethyl)imidazo[4,5-d]pyrrolo[2,3-b]pyridin-1(6H)-yl)piperidin-4-yl)ace tonitrile provided by the invention has the characteristics of good stability, high solubility and high bioavailability, and has broad application prospects. Of course, implementing any product or method of the present invention does not necessarily require achieving all the above-mentioned advantages simultaneously.

### DEFINITION AND DESCRIPTION

In the present invention, the term "substantially" used in "having an X-ray powder diffraction pattern substantially as shown in Figure 1" or "having an X-ray powder diffraction pattern essentially as shown in Figure 1" means that in the drawings the precise positions of the peaks should not be interpreted as absolute values. Because those skilled in the art know that the 2θ value of the X-ray powder diffraction pattern may cause errors due to different measurement conditions (such as the equipment and instruments used) and different samples, the measurement error of the diffraction angle of the X-ray powder diffraction pattern is 5% or less, typically ±0.2° difference from the given value is considered appropriate. It should also be understood that the relative intensity of the peaks may fluctuate with experimental conditions and sample preparation such as the preferred orientation of the particles in the sample. The use of automatic or fixed divergence slits will also affect the relative intensity calculation. The intensities shown in the XRPD curves included here are exemplary only and should not be used as an absolute comparison.

The water activity mentioned in the present invention refers to the ratio of the equilibrium vapor pressure of water in an ethanol aqueous solution to the saturated vapor pressure of pure water at the same temperature.

The present invention does not limit the drying temperature as long as the purpose of the present application can be achieved. For example, the drying temperature can be within the temperature range of 10°C to 30°C.

The therapeutically effective amount of a drug used in the present invention refers to a drug that is sufficient to affect the treatment of a disease, or at least one clinical symptom of a disease or condition when used in a treatment subject amount. The therapeutically effective amount may vary depending on the drug, the symptoms of the disease or condition, the severity of the symptoms of the disease or condition, and the like. In any event, a suitable dosage will be apparent to those skilled in the art or can be determined by routine experimentation.

The pharmaceutically acceptable carrier, diluent or excipient described in the present invention refers to a non-toxic carrier, diluent or excipient that will not adversely affect the pharmacological activity of the crystalline form formulated with it and is also safe for animal use excipient. Pharmaceutically acceptable carriers, diluents or excipients that can be used in the crystalline forms of the present invention include, but are not limited to, ion exchangers, aluminum oxide, aluminum stearate, magnesium stearate, lecithin, serum albumin, buffering substances such as phosphate, glycine, sorbic acid, potassium sorbate, a mixture of partial glycerides of saturated vegetable fatty acids, water, salt or electrolytes such as protamine sulfate, disodium hydrogen phosphate, potassium hydrogen phosphate, sodium chloride, zinc salt, silica gel, magnesium trisilicate, polyvinylpyrrolidone, cellulosic substances (such as microcrystalline cellulose, hydroxypropyl methylcellulose, lactose monohydrate, sodium lauryl sulfate, and croscarmellose sodium), polyethylene glycol, sodium carboxymethylcellulose, polyacrylate, wax, polyethylene-polypropylene oxide block polymer, polyethylene glycol and lanolin, etc.

Pharmaceutical compositions containing the crystalline form of the present invention may be administered orally, nasally inhaled, rectal, parenterally or topically. For oral administration, the pharmaceutical composition may be formulated into conventional solid dosage forms such as tablets, powders, granules, capsules, liquid dosage forms such as water or oil suspensions, or other forms such as syrups, solutions, suspensions. Liquid dosage form: when used for parenteral administration, the pharmaceutical composition can be made into a solution, aqueous solution, oily suspension, freeze-dried powder.

### DESCRIPTION OF FIGURE

In order to explain the embodiments of the present invention and the technical solutions of the prior art more clearly, the drawings needed to be used in the embodiments and the prior art are briefly introduced below. Obviously, the drawings in the following description are only for the purpose of explaining the embodiments of the present invention and the technical solutions of the prior art. For some embodiments of the invention, those of ordinary skill in the art can also obtain other drawings based on these drawings.
Figure 1 is the X-ray powder diffraction pattern of an amorphous compound;
Figure 2 X-ray powder diffraction pattern of crystalline form A;
Figure 3 Overlay of the differential scanning calorimetry spectrum and the thermogravimetric analysis spectrum of crystalline form A;
Figure 4 Three-dimensional structure diagram of crystalline form A;
Figure 5 X-ray powder diffraction pattern of crystalline form H;
Figure 6 Overlay of the differential scanning calorimetry spectrum and the thermogravimetric analysis spectrum of crystalline form H;
Figure 7 X-ray powder diffraction pattern of crystalline form S;
Figure 8 Overlay of the differential scanning calorimetry spectrum and the thermogravimetric analysis spectrum of crystalline form S;
Figure 9 X-ray powder diffraction pattern of crystalline form O;
Figure 10 Overlay of the differential scanning calorimetry spectrum and the thermogravimetric analysis spectrum of crystalline form O;
Figure 11 X-ray powder diffraction pattern of crystalline form P;
Figure 12 Overlay of differential scanning calorimetry spectrum and thermogravimetric analysis spectrum of crystalline form P;
Figure 13 X-ray powder diffraction pattern of crystalline form Q;
Figure 14 Polarizing microscope photo of crystalline form Q;
Figure 15 Overlay of differential scanning calorimetry spectrum and thermogravimetric analysis spectrum of crystalline form Q;
Figure 16 Proton NMR spectrum of crystalline form Q;
Figure 17 Dynamic gas phase adsorption diagram of crystalline form Q;
Figure 18 X-ray powder diffraction pattern of crystalline form M;
Figure 19 Overlay of differential scanning calorimetry spectrum and thermogravimetric analysis spectrum of crystalline form M;
Figure 20 Proton NMR spectrum of crystalline form M;
Figure 21 Changes in skin thickness in each group during the treatment of the atopic dermatitis animal model with crystalline form A;
Figure 22 Changes in the average auricle thickness of each group during the treatment of the atopic dermatitis animal model with crystalline form A;
Figure 23 Changes in skin lesion severity index severity index scores in each group during the treatment of atopic dermatitis animal models by crystalline form A;
Figure 24 Changes in skin thickness in each group during the treatment of psoriasis animal models with crystalline form A;
Figure 25 Changes in psoriasis area and severity index scores in each group during the treatment of psoriasis animal models with crystalline form A.

### ABBREVIATION:

XRPD: X-ray powder diffraction;
TGA: thermogravimetric analysis;
DSC: differential scanning calorimeter;
¹H-NMR: hydrogen nuclear magnetic resonance spectrum;
PLM: polarized light microscope;
DVS: dynamic vapor adsorption instrument;
SXRD: single crystal X-ray diffraction;
HPLC: high performance liquid chromatography;
LC-MS/MS: liquid chromatography tandem mass spectrometry;
ACN: acetonitrile;
DCM: dichloromethane;
n-heptane: n-heptane;
MeOH: methanol;
EtOH: ethanol;
EtOAc: ethyl acetate;
IPA: isopropyl alcohol;
2-MeTHF: 2-methyltetrahydrofuran;
2-Butanol: 2-butanol;
MEK: butanone;
THF: tetrahydrofuran;
MTBE: methyl tert-butyl ether;
FaSSIF: simulates fasting intestinal fluid;
SIF: simulated intestinal fluid;
FeSSIF: simulates eating intestinal fluid;
SGF: simulated gastric juice;
RH: relative humidity;
AD: atopic dermatitis;
SI: Skin lesion severity index;
PASI: Psoriasis Area and Severity Index;
Mean±SD: mean±standard deviation;
Mean±SEM: mean±standard error;
AAALAC: International Laboratory Animal Assessment and Accreditation Committee;
SPF: Specific Pathogen Free;
IVC: Independent Ventilation Cage.

### DETAILED IMPLEMENTATION

In order to make the purpose, technical solutions and advantages of the present invention more clear, the present invention will be further described in detail below with reference to the accompanying drawings and examples. Obviously, the described embodiments are only some of the embodiments of the present invention, but not all of the embodiments. Based on the embodiments of the present invention, all other embodiments obtained by those of ordinary skill in the art fall within the scope of protection of the present invention.

Unless otherwise stated, the detection instrument information and detection methods used in the present invention are as follows:

### (1) X-ray powder diffraction analysis

X-ray powder diffraction data of the samples were collected under ambient conditions using a Bruker D2 model X-ray powder diffractometer with an X-ray emitter power of 300W. The sample stage has no background signal, the step speed is 0.15 s/step, the total number of steps is 1837, the step length is 2θ = 0.02°, the voltage is 30 kV, and the current is 10 mA. The X-ray tube uses Cu target (Kα), and the Kα2/Kα1 intensity ratio is 0.50 (1.54439 Å/1.5406 Å).

### (2) Thermogravimetric analysis

Thermogravimetric data of the samples were collected using a TA Discovery series thermogravimeter TGA550. Put several milligrams of sample into a Tzero aluminum pan and heat it from room temperature to 300.0 °C under N₂ protection. The N₂ flow rate is 25 mL/min, and the heating rate is 10 °C/min.

### (3) Differential scanning calorimetry analysis

Thermal data of the sample was collected using a TA Discovery series differential scanning calorimeter DSC2500. Weigh several milligrams of sample into Tzero aluminum pans and seal with Tzero sealing caps. Heated to 300.0 °C under N₂ protection, N₂ flow rate was 50 mL/min, and heating rate was 10 °C/min.

### (4) Polarizing microscope

Use Olympus's BX3M-KMA-S polarizing microscope to observe the shape of the crystal form at room temperature and take PLM photos.

### (5) Dynamic gas phase adsorption instrument

The hygroscopicity data of the samples were collected using the ADVENTURE series dynamic gas phase adsorption instrument under N₂ protection. The sample dosage is about 30 mg.

The present invention will be described in detail below with reference to specific embodiments.

### Example 1: Preparation of amorphous form of compound of formula (I)

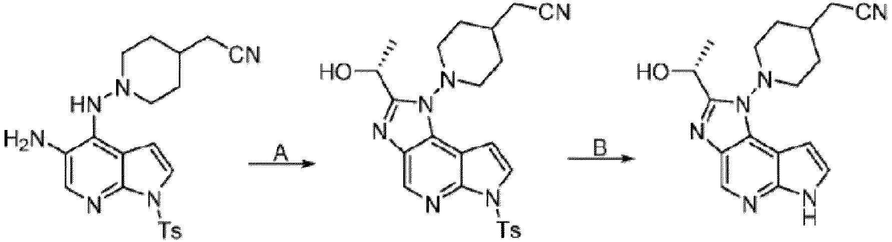

Step 1: Dissolve triethylboron tetrafluoride (251 mg, 1.32 mmol) and R-lactamide (118 mg, 1.32 mmol) in 8 mL tetrahydrofuran, stir and react at room temperature for 2 hours. Concentrate under reduced pressure to obtain a colorless oil, which was dissolved in 5 mL of absolute ethanol and added until the compound 2-(1-((5-amino-1-p-toluenesulfonyl-1H-pyrrolo[2,3-b]pyridin-4-yl)amino)piperidin-4-yl)acet onitrile (189 mg, 0.44 mmol) in 5 mL of absolute ethanol. The temperature was raised to 75°C and the reaction was stirred for 1 hour. After the reaction is completed, quench with sodium bicarbonate aqueous solution, add 30 mL of water, extract with ethyl acetate (3 × 30 mL), combine the organic phases, wash with 50 mL of saturated brine, and dry over anhydrous sodium sulfate. Suction filtration, concentrated filtrate under reduced pressure, silica gel column chromatography to obtain compound (R)-2-(1-(2-(1-hydroxyethyl)-6-p-toluenesulfonylimidazo[4,5-d]Pyrrolo[2,3-b]pyridin-1(6H)-yl)piperidin-4-yl)acetonitrile (110 mg, 52% yield). LCMS ESI (+) m/z: 479.1 (M+1).

Step 2: Compound (R)-2-(1-(2-(1-hydroxyethyl)-6-p-toluenesulfonylimidazo[4,5-d]pyrrolo[2,3-b]pyridine-1(6H) -yl)piperidin-4-yl)acetonitrile (110 mg, 0.23 mmol) was dissolved in 9 mL of methanol, 3 mL of 1N sodium hydroxide aqueous solution was added, and the reaction was stirred at room temperature for 16 hours. After the reaction is completed, adjust the pH to 7-8 with acetic acid, concentrate under reduced pressure, and prepare compound (R)-2-(1-(2-(1-hydroxyethyl)-imidazo[4,5-d]pyrrolo[2,3-b]pyridin-1(6H)-yl)piperidin-4-yl)ac etonitrile by silica gel column chromatography and high-performance liquid phase. (30 mg, 40% yield). ¹H NMR (400MHz, DMSO-d6) δ11.92 (s, 1H), 8.55 (s, 1H), 7.50 (d, J = 3.4Hz, 1H), 6.73 (d, J = 3.4Hz, 1H), 5.31 (s, 1H), 5.16 (q, J=6.6Hz, 1H), 3.65-3.53 (m, 2H), 3.15 (dd, J=30.2, 10.0Hz, 2H), 2.64 (d, J6.4Hz, 2H), 2.17-2.04 (m, 1H), 1.98-1.89 (m, 2H), 1.72-1.60 (m, 2H), 1.56 (d, J=6.6Hz, 3H). LCMS ESI (+) m/z: 325.0 (M+1). After determination, its XRPD pattern is shown in Figure 1, and the final product is amorphous.

(This preparation method has been recorded in page 11 of the specification of the compound patent WO2021051899A1 owned by the applicant.)

### Example 2: Preparation of crystalline form A of the compound of formula (I)

Weigh 15 mg of the amorphous substance of formula (I) in a 4 mL glass bottle, add ethanol to dissolve the sample, and place it into a 20 mL glass bottle containing 4 mL of n-heptane. Cap the 20 mL glass bottle tightly and place it at room temperature until solid precipitates. Separate the solid and determine it by XRPD, TGA, and DSC. The solid obtained is crystalline form A.

### Example 3: Preparation of crystalline form A of the compound of formula (I)

Dissolve 1.5kg of amorphous material of formula (I) in 15L of ethanol and stir at 75°C-85°C until the material is dissolved. Then concentrate to 1.5L-3L at 35°C-55°C, stir the obtained concentrated liquid at 5°C-15°C for 1-2 hours, and precipitate the solid; filter and dry to obtain 0.95kg solid, which is analyzed by XRPD, According to TGA and DSC measurements, the solid obtained was crystalline form A.

### Example 4: Preparation of crystalline form H of the compound of formula (I)

Weigh 15 mg of crystal form A sample, add 0.4 mL ACN, and perform magnetic stirring at room temperature to obtain a suspension. After about a week, the solid was separated and determined by XRPD, TGA, and DSC. The solid obtained was crystalline form H.

### Example 5: Preparation of crystalline form S of the compound of formula (I)

The crystal form Q (Example 8) was heated to 165 °C to obtain a solid, which was determined by XRPD, TGA, and DSC to be the crystalline form S.

### Example 6: Preparation of crystalline form O of the compound of formula (I)

Weigh 15 mg of crystal form A sample, add 0.4 mL DCM/n-heptane solution (2: 1, v/v), and perform magnetic stirring at room temperature to obtain a suspension. After about a week, the solid was separated, and the solid obtained was heated to 165 °C under N2 protection and then cooled to room temperature. The solid obtained was determined to be crystalline form O by XRPD, TGA, and DSC.

### Example 7: Preparation of crystalline form P of the compound of formula (I)

Weigh 15 mg of crystal form A sample, add MeOH solvent, and stir at room temperature to obtain a clear stock solution. Dispense the stock solution into 20 mL glass bottles. Under magnetic stirring conditions, gradually add 0.2 mL-0.5 mL of corresponding water to the glass bottles until a solid appears. Separate the solid and heat the solid to 120 °C to obtain a solid. XRPD, TGA, and DSC measurements showed that the solid obtained was crystalline form P.

### Example 8: Preparation of crystalline form Q of the compound of formula (I)

Stir crystal form A in an ethanol aqueous solution with a water activity of 0.6-0.8 until a solid is produced, centrifuge, and dry the collected solid. The solid obtained is determined by XRPD, TGA, and DSC to be crystalline form Q.

### Example 9: Preparation of crystalline form M of the compound of formula (I)

Weigh 15 mg of crystal form A sample, add EtOH solvent, and stir at room temperature to obtain a clear stock solution. Divide the stock solution into 20 mL glass bottles. Under magnetic stirring conditions, gradually add 0.2 mL-0.5 mL of the corresponding EtOAc solution into the glass bottles. Evaporate slowly at room temperature. After XRPD, TGA, and DSC measurements, the resulting solid is crystalline form M.

### Example 10: Single crystal X-ray diffraction (SXRD) experiment

Crystal preparation: weigh 15 mg of the crystal form A sample of the compound of formula (I) into a 15 mL test tube, add 1 mL of methylene chloride to dissolve, and then add 2 mL of ethanol. Cover the mouth of the test tube with a sealing film, use a needle to poke 5-10 small holes in the sealing film, and let it evaporate. After about three weeks, the solution evaporated completely, and colorless massive crystals were obtained at the bottom of the test tube.

Single crystal X-ray diffraction analysis was performed on the crystals. The results show that the crystal is a colorless block. The size of the crystal used in the diffraction analysis is 0.09×0.09×0.08mm3. It belongs to the monoclinic crystal system, the space group is P1211, and the unit cell parameters: a=9.6796(4)Å, α=90o, b =14.0338(6)Å, β=108.829.218(2) o, c=14.5745(6)Å, γ=90 o, unit cell volume V=1873.87(14)Å3, number of molecules in the unit cell Z=4, An independent area of the unit lattice contains one molecule.

### Example 11: Thermodynamic Analysis Experiment

Crystalline form H and crystalline form S were heated to appropriate temperatures under N₂ protection, and after cooling to room temperature, the XRPD of the solids was measured.

Results: 1) When crystalline form H was heated to 162 °C, the crystalline form H sample was partially converted into crystalline form A; when heated to 210°C, the sample was completely converted into crystalline form A. 2) Crystalline form S is heated to 215 °C, and the crystalline form S sample is converted into crystalline form A.

Analysis: 1) Combining the TGA/DSC spectrum of crystal form H, it can be seen that the weight loss of the sample heated to 210 °C is 0.6%, and DSC has two exothermic peaks before melting. According to the Burger-Ramberger rule, crystalline form H and crystalline form A have a monotropic relationship.

2) Combining the TGA/DSC spectrum of crystalline form S, it can be seen that the weight loss of the sample heated to 210 °C is 1.6%, and DSC has an exothermic peak before melting. According to the Burger-Ramberger rule, crystalline form S and crystalline form A have a monotropic relationship.

This experiment proves that compared with crystalline form H and crystalline form S, crystal form A is thermodynamically more stable.

### Example 12: Suspension competition experiment

Set up a suspension competition experiment using IPA and 2-MeTHF as solvents for crystalline form A, crystalline form O or crystalline form P at room temperature and 60 °C.

The specific operation is as follows: weigh 4 portions of excess crystalline form A into 4 HPLC vials, add 0.5 mL IPA to 2 portions, and add 0.5 mL 2-MeTHF to the other 2 portions. Place them at room temperature and 60 °C with magnetic stirring for about 1 hour respectively. Filter through a preheated nylon membrane (pore size 22 µm) to obtain a saturated solution; add 1 mg-2 mg each of crystalline form A, crystalline form O, and crystalline form P to the saturated filtrate, stir magnetically at room temperature or 60 °C for about 1 day, and centrifuge. Solids are used for XRPD testing. The results of the suspension competition experiment are shown in Table 3.

**Table 3 Result of suspension competition experiment**

| Starting crystal form | Solvent | Temperature (°C) | Obtained crystal form |
|---|---|---|---|
| crystal form A/ crystal form O/ crystal form P | IPA | Room temperature | Crystalline form A |
| | 2-MeTHF | | Crystalline form A |
| | IPA | 60 | Crystalline form A |
| | 2-MeTHF | | Crystalline form A |

The results show that the solids obtained in the two solvents at room temperature and 60 °C are all crystalline form A, indicating that compared with crystalline form O and crystalline form P, crystalline form A is more stable at room temperature and 60 °C.

### Example 13: Dynamic solubility

The dynamic solubility of crystalline form A and crystalline form Q in three biological solvents (simulated gastric juice, simulated fasting intestinal juice, and simulated eating intestinal juice) was tested at 37 °C to evaluate the solubility and stability of the two crystalline forms.

The specific operations for biosolvent preparation are as follows:
(1) Preparation method of FaSSIF: a. Weigh 42 mg NaOH, 395 mg NaH2PO4·H2O and 619 mg NaCl in a 100 mL beaker, add 90 mL deionized water to dissolve; b. Adjust the pH to 1N NaOH or 1N HCl. 6.5; Dilute to 100 mL with deionized water to obtain FaSSIF blank solvent; c. Weigh 224 mg of Simulated Intestinal Fluid (hereinafter referred to as SIF powder), add 50 mL of the above FaSSIF blank solvent, and stir until completely dissolved; use Dilute the FaSSIF blank solvent to 100 mL to prepare the solvent FaSSIF; d. Let it stand at room temperature for 2 hours before use.
(2) Preparation method of FeSSIF: a. Weigh 404 mg NaOH, 865 mg glacial acetic acid and 1189 mg NaCl in a 100 mL beaker, add 90 mL deionized water to dissolve; b. Adjust the pH to 5.0 with 1N NaOH or 1N HCl; Dilute to 100 mL with deionized water to obtain FeSSIF blank solvent; c. Weigh 1120 mg SIF powder and add 50 mL of the above FeSSIF blank solvent, stir until dissolved; dilute to 100 mL with FeSSIF blank solvent to obtain FeSSIF solvent ;d. Let it stand at room temperature before use to ensure a clear solution.
(3) Preparation method of SGF: a. Weigh 200 mg NaCl in a 100 mL beaker, add 90 mL deionized water to dissolve; b. Adjust the pH to 1.6 with 1N HCl; dilute to 100 mL with deionized water to obtain SGF Blank solvent; c. Weigh 6 mg SIF powder and add 50 mL of the above SGF blank solvent, stir until dissolved; dilute to 100 mL with SGF blank solvent to prepare the solvent SGF; d. Let it stand at room temperature before use to ensure a clear solution .

The specific method of dynamic solubility testing is as follows: add approximately 40 mg of crystalline form A or crystalline form Q to 4 mL of three biological solvents respectively, and shake at a constant temperature of 37 °C (100 rpm). Take 1.3 mL of the suspension (or clarified solution) respectively at 1, 4, and 24 hours, and centrifuge the suspension for 5 minutes. The solid obtained is used for crystal form detection (XRPD), and the supernatant/clarified solution is filtered for solubility testing and pH test.

Solubility data of the samples were collected using a high-performance liquid chromatograph equipped with an image diode array detector.

Test results: The dynamic solubility test results are shown in Table 4.

**Table 4 Measurement results of dynamic solubility**

| Dynamic solubility in SGF at 37 °C | | | | | | | | | |
|---|---|---|---|---|---|---|---|---|---|
| Sample | 1h | | | 4h | | | 24h | | |
| | Solubility | pH | Changes in Crystal form | Solubility | pH | Changes in Crystal form | Solubility | pH | Changes in Crystal form |
| Crystalline form A | 0.72 | 2.92 | no change | 0.79 | 2.99 | -- | 0.82 | 2.95 | no change |
| Crystalline form Q | 0.62 | 2.60 | no change | 0.69 | 2.98 | -- | 0.67 | 2.71 | no change |

| Dynamic solubility in FaSSIF at 37 °C | | | | | | | | | |
|---|---|---|---|---|---|---|---|---|---|
| Sample | 1h | | | 4h | | | 24h | | |
| | Solubility | pH | Changes in Crystal form | Solubility | pH | Changes in Crystal form | Solubility | pH | Changes in Crystal form |
| Crystalline form A | 0.05 | 6.41 | no change | 0.06 | 6.49 | -- | 0.05 | 6.54 | no change |
| Crystalline form Q | 0.03 | 6.45 | no change | 0.04 | 6.40 | -- | 0.03 | 6.49 | no change |

| Dynamic solubility in FeSSIF at 37 °C | | | | | | | | | |
|---|---|---|---|---|---|---|---|---|---|
| Sample | 1h | | | 4h | | | 24h | | |
| | Solubility | pH | Changes in Crystal form | Solubility | pH | Changes in Crystal form | Solubility | pH | Changes in Crystal form |
| Crystalline form A | 0.08 | 4.92 | no change | 0.08 | 4.95 | -- | 0.09 | 4.99 | no change |
| Crystalline form Q | 0.05 | 4.96 | -- | 0.04 | 4.98 | -- | 0.05 | 5.00 | no change |

| | | | | | | | | | |
|---|---|---|---|---|---|---|---|---|---|
| Note: Starting sample: Crystalline form A, Crystalline form Q --: The crystal form of the 24-hour undissolved sample is the same as the starting sample, so the crystal form of the 1-hour or 4-hour undissolved sample was not tested. | | | | | | | | | |

The results show that: within 24 hours at 37 °C, 1) the solubility of crystalline form A in three solvents (SGF/FaSSIF/FeSSIF) is greater than that of crystalline form Q; 2) the solubility of crystalline form A and crystalline form Q in SGF/FaSSIF/ Suspended and stirred in FeSSIF for 24 hours, the crystal forms were stable.

### Example 14: Solid state stability

Test method: Weigh 17-20 mg of crystalline form A and crystalline form Q samples respectively into HPLC vials, and place them open at 1) 25 °C/60%RH, 2) 40 °C/75%RH, 3) 60 °C under certain conditions. Purity testing (via HPLC) and crystal form detection (via XRPD) were performed on starting samples, samples stored for one week, and samples stored for two weeks. The specific results are shown in Table 5.

**Table 5 2-week stability study data of crystalline form A and crystalline form Q**

| Starting sample | | Condition | 1 week | | 2week | |
|---|---|---|---|---|---|---|
| Crystalline form | Purity (%) | | Purity (%) | Crystal form changes | Purity (%) | Crystal form changes |
| Crystalline form A | 99.59 | 25°C/60%RH | 99.64 | no change | 99.60 | no change |
| | | 40 °C/75%RH | 99.62 | | 99.60 | |
| | | 60 °C | 99.62 | | 99.66 | |
| Crystalline form Q | 99.87 | 25 °C/60%RH | 99.80 | no change | 99.87 | no change |
| | | 40 °C/75%RH | 99.93 | | 99.87 | |
| | | 60 °C | 99.91 | | 99.92 | |

The results show: 1) Purity: After one week and two weeks under the selected conditions, there is no significant change in the purity of crystalline form A and crystalline form Q. 2) Changes in crystal form: The XRPD comparison results of crystal forms A and Q stored under three different conditions for 0, 1, and 2 weeks show that after one week and two weeks of storage under the selected conditions, there is no change in the crystalline form.

Conclusion: Both crystalline form A and crystalline form Q have good solid-state stability.

### Example 15: Crystal form stability test

The crystalline form M was stored at a temperature of 25 °C and a humidity of 55% RH for 35 days, and the XRPD of the solid was tested. After detection, the crystalline form M was transformed into the crystalline form A.

The results show that under ambient temperature and humidity, crystalline form M is unstable and easily transforms into stable crystalline form A.

### Example 16: Hygroscopicity experiment

The hygroscopicity of the above crystalline form was determined with reference to the 2020 edition of the Chinese Pharmacopoeia, General Chapter 9103, Guidelines for Drug Hygroscopicity Tests.

The criteria for judging hygroscopicity are:
Deliquescence: Absorbing enough water to form a liquid;
Extremely hygroscopic: the weight gain by attracting moisture is not less than 15%;
It has hygroscopicity: the weight gain by attracting moisture is less than 15%, but not less than 2%;
Slightly hygroscopic: weight gain due to moisture attraction is less than 2%, but not less than 0.2%;
No or almost no hygroscopicity: weight gain due to moisture absorption is less than 0.2%.

It can be seen from the test results that the weight gain of crystalline form A is 0.05%, which means it has no or almost no hygroscopicity. Compared with other crystal forms recorded in this patent, it is more suitable for the preparation of solid preparations.

### Example 17: Influencing factors test

The stability of the crystalline form of the present invention under accelerated test conditions was determined. Place crystalline form A, crystalline form H, crystalline form S, crystalline form O, crystalline form P, crystalline form Q and crystalline form M respectively in a 5000Lx light environment, a 60°C high temperature environment and a 90%RH±5%RH high humidity environment. Stability testing was conducted under the following conditions, and samples were taken on the 0th day, the 5th day, the 10th day and the 30th day. The content, total impurities, and XRPD patterns of the samples were recorded and compared with the initial data. The comparison results show that crystalline form A, crystalline form H, crystalline form S, crystalline form O, crystalline form P, crystalline form Q and crystalline form M have better stability, especially crystalline form A has better stability. The experimental data of influencing factors of crystalline form A are shown in Table 5a below:

**Table 5a Experimental data of influencing factors of crystalline form A**

| Inspection project | 0d | High temperature test | | | High humidity test | | | Light test | | |
|---|---|---|---|---|---|---|---|---|---|---|
| | | (60°C) | | | (25°C, 90±5%RH) | | | (Illuminance 5000Lx and UV lamp energy 0.9W/m²) | | |
| | | 5d | 10d | 30d | 5d | 10d | 30d | 5d | 10d | 30d |
| Total impurity | 0.325% | 0.34% | 0.33% | 0.35% | 0.32% | 0.31% | 0.32% | 0.34% | 0.36% | 0.43% |
| Content (%) | 99.1% | 100.2% | 100.4% | 100.3% | 100.1% | 100.1% | 100.1% | 99.5% | 98.9% | 99.4% |
| XRPD Test | Crystal form A | Crystal form A | Crystal form A | Crystal form A | Crystal form A | Crystal form A | Crystal form A | Crystal form A | Crystal form A | Crystal form A |

### Example 18: Pharmacokinetic experiment

12 SD rats were divided into two groups, with 6 rats in each group, half male and half female, and each was given a single intragastric administration; blood was collected through the fundus venous plexus at designated time points, and the plasma was separated and stored in a -80°C refrigerator.

For the above plasma sample, after protein precipitation with acetonitrile, the supernatant was diluted with water and 5 µL was transferred to LC-MS/MS for detection.

It was found that the crystalline form A sample has high bioavailability in the body and meets the needs of drug development.

### Pharmacological efficacy experiments

### Example A Pharmacological effects in animal models of atopic dermatitis.

### 1. Experimental materials

### (1) Experimental animals and feeding methods

BALB/C mice, 55 pieces, female, were 6 weeks old at the time of purchase and 8 weeks old at the time of experiment, weighing 18g-20g. The animals were from Shanghai Lingchang Biotechnology Co, Ltd (SCXK (Shanghai) 2018-0003), experimental animal production certificate number 20180003012342; experimental animal use license number: SYXK(Shanghai)2020-0001(Via Biotechnology (Shanghai) Ltd.).

All mice were kept in an IVC constant temperature and pressure system in an SPF-grade animal room, with a temperature of 20°C-26°C, a humidity of 40%-70%, and a photoperiod of 12 hours light and 12 hours dark. Six mice were kept in each cage, the size of the cage was 325 mm × 210 mm × 180 mm, and the bedding in the cage was changed twice a week. During the entire experiment, all experimental mice had free access to food and drink, and the feed and water were autoclaved and replaced twice a week.

### (2) Test sample

a. Tofacitinib, free base, purity is 98%, total amount is 200 mg, storage temperature is 4°C.
b. Upadacitinib, free base, purity is 98%, total amount is 70mg, storage temperature is 4°C.
c. Crystalline form A, free base, purity is 99.7%, total amount is 300 mg, storage temperature is 4°C.

### 2. Experimental procedures and methods

Modeling and administration method: Use depilatory cream to remove the hair of an area of about 5cm × 2cm from the top of the head to the back of each animal. The application time of the depilatory cream should be controlled within 2 minutes. Wash the depilated area with warm water and wash off the depilatory agent, immediately use a hair dryer to dry the animal's hair, and place the animal on a 37°C constant-temperature electric blanket for 1h-2h. After the animal moves freely, place it in a breeding cage and recover for 1 day (if the animal's skin is red, swollen and damaged after hair removal, it will its elimination). On the second day, 42 animals with normal skin were selected and randomly divided into groups according to body weight, with 6 animals in each group, totaling 7 groups. Except for the G1 normal control group, each animal in the other groups was evenly smeared with 200 µL of 0.5wt% dinitrochlorobenzene solution (the solvent was acetone and olive oil with a mass ratio of 3:1) on the back and auricle for three consecutive days every day. Induction (the first stage of induction), the second stage of induction started after 14 days, and was induced with 1wt% dinitrochlorobenzene solution (the solvent was acetone and olive oil with a mass ratio of 3:1) every three days until the end of the experiment, start treatment and administration at the same time, and group administration is shown in Table 6.

**Table 6 Administration route, dosage and grouping of AD model drug efficacy experiments**

| Group | Number of animals | dinitrochlorobenzene | Administration group | Dosage (mg/kg) | Administration route | dosing frequency |
|---|---|---|---|---|---|---|
| 1 | 6 | - | Blank control group | - | - | - |
| 2 | 6 | + | Negative control | - | Oral | Twice a day * 14 days |
| 3 | 6 | + | Tofacitinib | 30 | Oral | Twice a day * 14 days |
| 4 | 6 | + | Upatinib | 10 | Oral | Twice a day * 14 days |
| 5 | 6 | + | Crystalline form A | 3 | Oral | Twice a day * 14 days |
| 6 | 6 | + | Crystalline form A | 10 | Oral | Twice a day * 14 days |
| 7 | 6 | + | Crystalline form A | 30 | Oral | Twice a day * 14 days |

The preparation method of the test drug is shown in Table 7.

**Table 7 Preparation Method of Test Drugs**

| Sample name | Preparation method | Dosage concentration (mg/mL) | storage |
|---|---|---|---|
| Tofacitinib | 0.5 g of methylcellulose+0.25 mL of Tween 20, add sterile deionized water to 100 mL, then add 300 mg of tofacitinib and sonicate until fully dissolved | 3mg/mL, twice a day | 4°C |
| Crystalline form A | Take three parts of 0.5 g methylcellulose, add sterile deionized water to 100 mL, and then add 300 mg, 100 mg, and 30 mg of crystalline form A separately. Shake with ultrasound until fully dissolved | 3mg/mL, 1mg/mL, 0.3mg/mL, Twice a day | 4°C |
| Upatinib | 0.5 g of methylcellulose+0.25 mL of Tween 20, add sterile deionized water to 100 mL, then add 100 mg of Upatinib and sonicate until fully dissolved | 1mg/mL, twice a day | 4°C |

### 3. Experimental observation

During the entire experimental process, the use and observation of experimental animals were carried out in accordance with the relevant regulations of AAALAC on the use and management of animals.

### 4. Evaluation indicators

Skin thickness (double layer): Use a vernier caliper to measure the double layer thickness of the back skin on the 1 day before modeling, and on the 14th, 17th, 20th, 23rd, 26th, and 28th days after modeling.

Auricle thickness: Use a vernier caliper to measure the thickness of the right auricle 1 day before modeling, and on days 14, 17, 20, 23, 26, and 28 after modeling.

AD-SI score: The condition score refers to the clinical SI scoring standard, and scores three indicators: skin color/erythema, scaly erythema, scaly/peeling/lichenification, thickening, and thickening/edema degree. The total score is 12 points. Scoring standard as follows:
Erythema/skin color: 0 points, none; 1 points, slight: light red; 2 points, moderate: red; 3 points, significant: dark red; 4 points, very obvious: extremely deep red.

Scaling/peeling/lichenification: 0 points, none; 1 point, slight: there are scales/peeling/lichenification on part of the epidermis, mainly subtle; 2 points, moderate: most of the skin surface is completely or incompletely covered with Scaling/peeling/lichenification, flaky; 3 points, significant: almost all skin is covered with scales/peeling/lichenification, and the scales/peeling/lichenification are thick and layered; 4 points, very obvious: all skin is covered with Scaling/peeling/lichenification, very thick and layered.

Thickening/edema: 0 points, none: flush with normal skin; 1 points, slight: slightly higher than the normal skin surface; 2 points, moderate: moderately raised, with round or sloped plaque edges; 3 points, obvious Score: The plaque is hypertrophic and the bulge is obvious; 4 points, very significant: it is highly thickened and the bulge is very obvious.

Skin/auricle hyperplasia inhibition rate (%) = 1-[(skin/auricle thickness of the treatment group - skin/auricle thickness of the blank control group)/skin/auricle thickness of the negative control group - skin/auricle of the blank control group thickness)]%.

### 5. Experiment termination

The experiment was ended after 14 days of treatment, and the animals were euthanized. The back skin and auricles of the mice were dissected and separated, half of which were fixed and stored in formalin, and the other half were stored at -80°C.

### 6. Data analysis

All data are expressed as Mean ± SD or Mean ± SEM. Use the statistical analysis software SPSS23.0 to conduct normal distribution and homogeneity of variance tests on the data. Select and homogeneity of variance tests based on the results, and select the one-factor analysis of variance method based on the results.*P<0.05 will be considered a statistically significant difference.

### 7. Experimental results

### (1) Effect of tested drugs on skin thickness

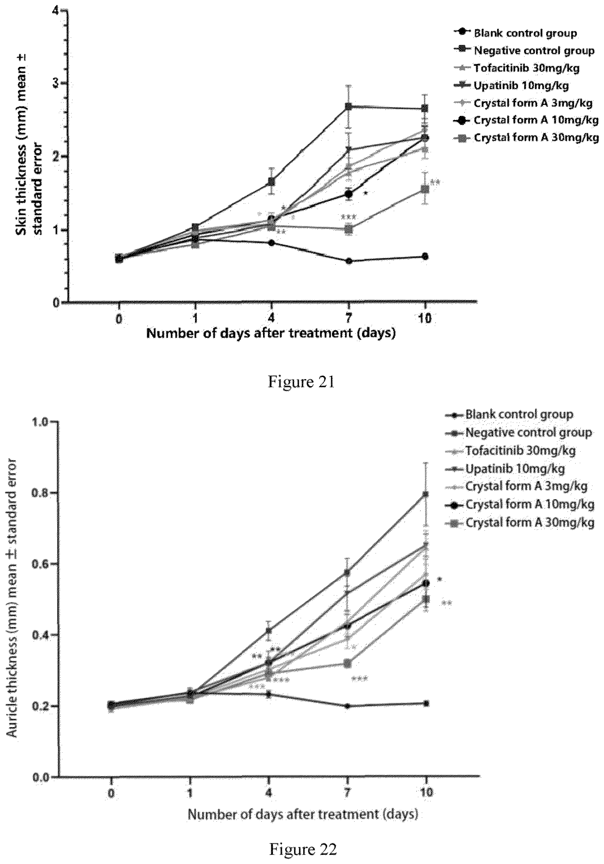

On the 10th day of treatment, the average skin thickness changes and inhibitory effects of each group are shown in Table 8, Table 9 and Figure 21. Figure 21 shows the changes in skin thickness of each group during the treatment period (Note: *P<0.05, **P<0.01, ***P<0.001 are all compared with the G2 negative control group).

**Table 8 Average skin thickness (double layer) change data (Mean±SEM) (n=6)**

| Group | Average skin thickness (double layer) (mm) | | | | |
|---|---|---|---|---|---|
| | Day 0 | Day 1 | Day 4 | Day 7 | Day 10 |
| G1 blank control group | 0.61±0.01 | 0.87±0.02 | 0.81±0.02 | 0.57±0.01 | 0.63±0.01 |
| G2 negative control group | 0.60±0.02 | 1.04±0.02 | 1.66±0.17 | 2.67±0.3 | 2.64±0.2 |
| G3 Tofacitinib 30mg/kg | 0.63±0.03 | 0.99±0.03 | 1.13±0.1* | 1.78±0.11 | 2.08±0.13 |
| G4 Upatinib 10mg/kg | 0.60±0.02 | 0.89±0.04 | 1.07±0.05* | 2.06±0.25 | 2.23±0.14 |
| G5 Crystalline form A 3mg/kg | 0.63±0.01 | 0.98±0.01 | 1.08±0.01** | 1.85±0.12 | 2.35±0.15 |
| G6 Crystalline form A 10mg/kg | 0.60±0.02 | 0.94±0.02 | 1.14±0.04 | 1.49±0.09* | 2.23±0.17 |
| G7 Crystalline form A 30mg/kg | 0.62±0.02 | 0.79±0.05 | 1.05±0.03* * | 1.01±0.08* ** | 1.56±0.21 * |

| | | | | | |
|---|---|---|---|---|---|
| Note: *P<0.05, **P<0.01, ***P<0.001 are compared with G2 negative control group | | | | | |

**Table 9 Inhibition of skin thickening in each treatment group on the 10th day of treatment (n=6)**

| Group | Average skin thickness on day 10 (mm) | Inhibition rate | P value |
|---|---|---|---|
| G1 blank control group | 0.63 | / | |
| G2 negative control group | 2.64 | / | |
| G3 Tofacitinib 30mg/kg | 2.08 | 27.86% | 0.118 |
| G4 Upatinib 10mg/kg | 2.23 | 20.40% | 0.390 |
| G5 Crystalline form A 3mg/kg | 2.35 | 14.43% | 0.580 |
| G6 Crystalline form A 10mg/kg | 2.23 | 20.40% | 0.312 |
| G7. Crystalline form A 30mg/kg | 1.56 | 53.73% | 0.007** |

| | | | |
|---|---|---|---|
| Note: *P<0.05, **P<0.01, ***P<0.001 are compared with G2 negative control group | | | |

### (2) Effect of tested drugs on auricle thickness

On the 10th day of treatment, the average auricle thickness changes and inhibitory effects of each group are shown in Table 10, Table 11 and Figure 22. Figure 22 shows the changes in the average auricle thickness of each group during the treatment period (Note: *P<0.05, **P<0.01, ***P<0.001 are all compared with the G2 negative control group).

**Table 10 Average auricle thickness data (Mean±SEM) (n=6)**

| Group | Average skin thickness (double layer) (mm) | | | | |
|---|---|---|---|---|---|
| | Day 0 | Day 1 | Day 4 | Day 7 | Day 10 |
| G1 blank control group | 0.21±0.00 | 0.24±0.00 | 0.23±0.01 | 0.20±0.00 | 0.21±0.01 |
| G2 negative control group | 0.20±0.00 | 0.23±0.00 | 0.41±0.03 | 0.58±0.04 | 0.79±0.09 |
| G3 Tofacitinib 30 mg/kg | 0.19±0.01 | 0.22±0.01 | 0.28±0.01 *** | 0.44±0.03 | 0.64±0.05 |
| G4 Upatinib 10 mg/kg | 0.20±0.01 | 0.24±0.01 | 0.32±0.03 ** | 0.51±0.05 | 0.65±0.03 |
| G5 Crystalline form A 3 mg/kg | 0.20±0.00 | 0.22±0.01 | 0.30±0.01 *** | 0.39±0.03* | 0.57±0.04 |
| G6 Crystalline form A 10 mg/kg | 0.20±0.01 | 0.23±0.01 | 0.32±0.01 ** | 0.43±0.03 | 0.54±0.07* |
| G7 Crystalline form A 30 mg/kg | 0.21±0.01 | 0.22±0.01 | 0.29±0.02 *** | 0.32±0.01*** | 0.50±0.03* * |

| | | | | | |
|---|---|---|---|---|---|
| Note: * P<0.05, * * P<0.01, * * * P<0.001 are all compared with the G2 negative control group | | | | | |

**Table 11: Inhibition of auricular thickening in each treatment group on the 10th day of treatment(n=6)**

| Group | Average skin thickness on the 10th day (mm) | Inhibition rate | P value |
|---|---|---|---|
| G1 blank control group | 0.21 | / | |
| G2 negative control group | 0.79 | / | |
| G3 Tofacitinib 30 mg/kg | 0.64 | 25.86% | 0.289 |
| G4 Upatinib 10 mg/kg | 0.65 | 24.14% | 0.410 |
| G5 Crystalline form A 3 mg/kg | 0.57 | 37.93% | 0.056 |
| G6 Crystalline form A 10 mg/kg | 0.54 | 43.10% | 0.035* |
| G7 Crystalline form A 30 mg/kg | 0.50 | 50.00% | 0.006** |

| | | | |
|---|---|---|---|
| Note: *P<0.05, **P<0.01, ***P<0.001 are compared with G2 negative control group | | | |

### (3) Effect of test drugs on AD-SI scores in AD model animals

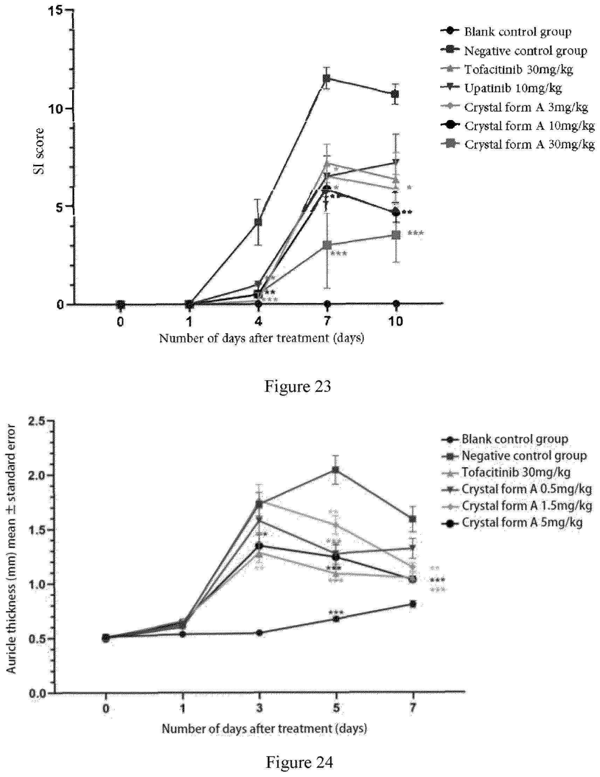

As of the 10th day of treatment, the changes in AD-SI scores in each group (n=6) are shown in Table 12 and Figure 23. Figure 23 shows the changes in AD-SI scores in each group during treatment.

**Table 12 AD-SI score data during treatment**

| Group | AD-SI score (Mean±SD) | | | | |
|---|---|---|---|---|---|
| | Day 0 | Day 1 | Day 4 | Day 7 | Day 10 |
| G1 blank control group | 0.00 | 0.00 | 0.00 | 0.00 | 0.00 |
| G2 negative control group | 0.00 | 0.00 | 4.17±1.17 | 11.50±0.55 | 10.67±0.52 |
| G3 Tofacitinib 30 mg/kg | 0.00 | 0.00 | 0.17±0.41*** | 7.17±0.98 | 6.33±1.37 |
| G4 Upatinib 10 mg/kg | 0.00 | 0.00 | 1.00±0.00 | 6.50±1.05* | 7.17±1.47 |
| G5 Crystalline form A 3 mg/kg | 0.00 | 0.00 | 1.00±0.00 | 6.50±1.05* | 5.83±0.75* |
| G6 Crystalline form A 10 mg/kg | 0.00 | 0.00 | 0.50±0.55** | 5.83±1.17** | 4.67±0.52** |
| G7 Crystalline form A 30 mg/kg | 0.00 | 0.00 | 0.50±0.55** | 3.00±2.19*** | 3.50±1.39*** |

| | | | | | |
|---|---|---|---|---|---|
| Note: *P<0.05, **P<0.01, ***P<0.001 are compared with G2 negative control group | | | | | |

**Result analysis**: On the 10th day of treatment, the average back double-layer skin thickness of the G7 group treated with crystalline form A (30 mg/kg, twice a day) was 1.56mm, the average auricle thickness was 0.5mm, and the average score was 3.5 points , compared with the G2 negative control group (the average back double-layer skin thickness was 2.64mm, the average auricle thickness was 0.79mm, and the average AD-SI score was 10.67 points), it significantly inhibited the inflammatory thickening of the back skin and significantly improved AD symptoms. As for the effect of performance, the inhibition rate of back skin thickness was 53.73%, and the inhibition rate of auricle thickness was 50%, and there were statistically significant differences (P<0.01).

On the 10th day of treatment, the average auricle thickness of the G6 group treated with crystalline form A (10 mg/kg, twice a day) was 0.54 mm, and the average AD-SI score was 4.67 points, which was the same as the G2 negative control group (auricle Compared with the average thickness of 0.79mm and the average AD-SI score of 10.67 points), it can significantly inhibit the inflammatory thickening of the auricle and improve the performance of AD symptoms. The auricle thickness inhibition rate is 43.1%, and there is a statistically significant Difference in penetration (P<0.05); the average back double-layer skin thickness of the crystalline form A (10 mg/kg, twice a day) treatment group was 2.23mm. Compared with the G2 negative control group, it inhibited the inflammatory thickening of the back skin. effect, the inhibition rate was 20.4%.

### Example B: Pharmacodynamic effects in psoriasis animal models.

### 1. Experimental materials

### (1) Experimental animals and feeding methods

C57/BL 6J mice, 75, female, 7-8 weeks old, weight 18g-20g. The animals were from Zhejiang Weitong Lihua Experimental Animal Technology Co., Ltd. (SCXK (Zhejiang) 2019-0001), experimental animal production certificate number 20210107Abzz0619000247; experimental animal use license number: SYXK (Shanghai) 2020-0001 (Via Biotechnology (Shanghai Co., Ltd).

All mice were kept in an IVC constant temperature and pressure system in an SPF-grade animal room, with a temperature of 20°C-26°C, a humidity of 40%-70%, and a photoperiod of 12 hours light and 12 hours dark. 3-5 mice were raised in each cage, the size of the cage was 325 mm × 210 mm × 180 mm, and the bedding in the cage was changed twice a week. During the entire experiment, all experimental mice had free access to food and drink, and the feed and water were autoclaved and replaced twice a week.

### (2) Test sample

a. Tofacitinib, free base, purity is 98%, total amount is 100 mg, storage temperature is 4°C.
b. Crystalline form A, purity is 99.7%, total amount is 200 mg, storage temperature is 4°C.

### 2. Experimental procedures and methods

Modeling and administration method: Use depilatory cream to remove hair from an area of about 2cm × 3cm on the back of each animal. Control the application time of depilatory cream within 2 minutes. Wash the depilated area with warm water, wash off the depilatory agent, and use immediately. Dry the animal's hair with a hair dryer, and place the animal on a constant-temperature electric blanket at 37°C for 1h-2h. After the animal moves freely, it is placed in a cage for normal rearing and recovery for 1 day (if any animal has red, swollen and damaged skin after hair removal, remove it) . On the second day, 54 animals with normal skin were selected and randomly grouped according to body weight, with 9 animals in each group, a total of 6 groups. Each animal in the G2-G6 group was evenly applied with 60 mg (60 µL) of 5wt% imiquimod cream (Aldara) on the back every day for seven consecutive days. The administration route, dosage and grouping of the psoriasis model drug efficacy experiment are shown in Table 13.

**Table 13 Administration route, dosage and grouping of drug efficacy experiments in psoriasis model**

| Group | Number of animals | Imiquimod cream | Dosing group | Dosage (mg/kg) | Route of administration | Dosing frequency |
|---|---|---|---|---|---|---|
| 1 | 9 | - | Blank control group | - | - | - |
| 2 | 9 | + | Negative control group | - | oral | twice a day*7 |
| 3 | 9 | + | tofacitinib | 30 | oral | twice a day*7 |
| 4 | 9 | + | Crystalline form A | 0.5 | oral | twice a day*7 |
| 5 | 9 | + | Crystalline form A | 1.5 | oral | twice a day*7 |
| 6 | 9 | + | Crystalline form A | 5 | oral | twice a day*7 |

Preparation method of test drugs: as shown in Table 14.

**Table 14 Preparation methods of test drugs**

| sample name | Preparation method | Dosing concentration (mg/mL) | store |
|---|---|---|---|
| tofacitinib | 0.5g methylcellulose + 0.25mL Tween 20, Add sterile deionized water to 100 mL, then add 600 mg tofacitinib and shake with ultrasonic until completely dissolved. | 6mg/mL, 2 times a day | 4°C |
| Crystalline form A | Take three portions of 0.5 g methylcellulose, | 0.1mg/mL, 0.3mg/mL, 1mg/mL, 2 times a day | 4°C |
| | Add sterile deionized water to 100 mL, then add 10 mg, 30 mg, and 100 mg respectively. | | |
| | Crystal form A is sonicated until completely dissolved | | |

### 3. Experimental observation

During the entire experimental process, the use and observation of experimental animals were carried out in accordance with the relevant regulations of AAALAC on the use and management of animals.

### 4. Evaluation indicators

Skin thickness (double-layer): Use a vernier caliper to measure the double-layer thickness of the back skin before drug administration and modeling, and on days 1, 3, 5, and 7 after drug administration.

PASI score: The condition score refers to the clinical PASI scoring standard, and scores three indicators: erythema, scale, and thickness. The total score is 12 points. The scoring standards are as follows:
Erythema: 0 points, none; 1 points, slight: light red; 2 points, moderate: red; 3 points, significant: dark red; 4 points, very obvious: extremely deep red.

Scaling: 0 points, none; 1 point, slight: there are scales on part of the epidermis, mainly fine scales; 2 points, moderate: most of the skin surface is completely or incompletely covered with scales, in a flaky form; 3 points, Significant: almost all skin is covered with scales, and the scales are thick and layered; 4 points, very obvious: all the skin is covered with scales, and the scales are thick and layered.

Plaque thickening degree: 0 points, none: flush with normal skin; 1 points, slight: slightly higher than the skin surface; 2 points, moderate: moderately raised, with round or sloped plaque edges; 3 points, obvious Score: The plaque is hypertrophic and the bulge is obvious; 4 points, very significant: it is highly thickened and the bulge is very obvious.

Skin proliferation inhibition rate (%) = 1 - [(skin thickness of the treatment group on the 7th day - skin thickness of the blank control group on the 7th day)/skin thickness of the negative control group on the 7th day - skin thickness of the blank control group on the 7th day)] %.

After the experiment, the mouse back skin was dissected and separated, fixed and preserved in formalin.

### 5. Experiment termination

After 7 days of treatment, the animals were euthanized, the mouse back skin was dissected and separated, and fixed and preserved in formalin.

### 6. Data analysis

All data are expressed as Mean ± SEM. The statistical analysis software SPSS23.0 was used to conduct normal distribution and homogeneity of variance tests on the data. Based on the results, the single-factor analysis of variance method was selected and the homogeneity of variance test was selected based on the results. *P<0.05 will be considered a statistically significant difference.

### 7. Experimental results

### (1) Effect of tested drugs on skin thickness

On the 7th day of treatment, the average skin thickness changes and inhibitory effects of each group are shown in Table 15, Table 16 and Figure 24. Figure 24 shows the changes in skin thickness of each group during treatment.

**Table 15 Average skin thickness (double layer) change data (Mean±SEM) (n=9)**

| Group | Average skin thickness (double layer) (mm) | | | | |
|---|---|---|---|---|---|
| | Day 0 | Day 1 | Day 3 | Day 5 | Day 7 |
| G1 blank control group | 0.51±0.01 | 0.54±0.01 | 0.54 ±0.01 | 0.67 ±0.02 | 0.81±0.03 |
| G2 negative control group | 0.51±0.01 | 0.62±0.02 | 1.73 ±0.11 | 2.04 ±0.13 | 1.59±0.12 |
| G3. Tofacitinib 30mg/kg | 0.51±0.01 | 0.66±0.02 | 1.28±0.09** | 1.09±0.06*** | 1.05±0.04*** |
| G4. Crystalline form A 0.5mg/kg | 0.50±0.01 | 0.60±0.01 | 1.57 ±0.11 | 1.27+0.1 *** | 1.32±0.09 |
| G5. Crystalline form A 1.5mg/kg | 0.50±0.01 | 0.61±0.02 | 1.76 ±0.15 | 1.53±0.08** | 1.15±0.04** |
| G6. Crystalline form A 5mg/kg | 0.50±0.01 | 0.64±0.01 | 1.35 ±0.10* | 1.24±0.11*** | 1.03±0.03*** |

| | | | | | |
|---|---|---|---|---|---|
| Note: *P<0.05, **P<0.01, ***P<0.001 are compared with G2 negative control group | | | | | |

**Table 16 Inhibition of skin thickening in each treatment group on the 7th day of treatment (n=9)**

| Group | Average skin thickness on the 7th day (mm) | Inhibition rate | P value |
|---|---|---|---|
| G1 blank control group | 0.81 | / | |
| G2 negative control group | 1.59 | / | |
| G3. Tofacitinib 30 mg/kg | 1.05 | 69.2% | 0.000 |
| G4. Crystalline form A 0.5 mg/kg | 1.32 | 34.6% | 0.159 |
| G5. Crystalline form A 1.5 mg/kg | 1.15 | 56.4% | 0.017 |
| G6. Crystalline form A 5 mg/kg | 1.03 | 71.8% | 0.000 |

| | | | |
|---|---|---|---|
| Note: * P<0.05, * * P<0.01, * * * P<0.001 are all compared with the G2 negative control group | | | |

### (2) The effect of the test drug on the Disease Severity Index (PASI) score in psoriasis model animals

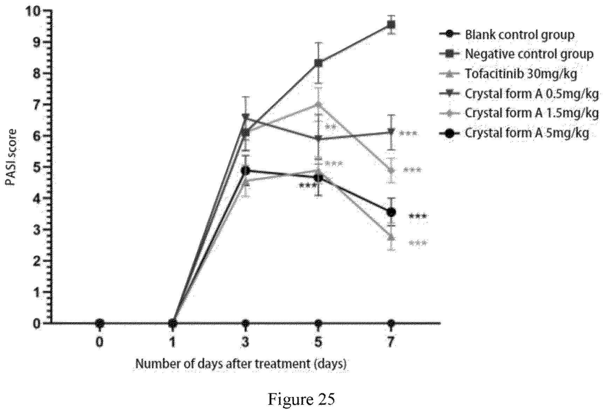

On the 7th day of treatment, the changes in PASI scores in each group (n=9) are shown in Table 17 and Figure 25. Figure 25 shows the changes in PASI scores among different groups during the treatment period.

**Table 17 PASI score data during treatment**

| Group | PASI score (Mean ± SEM) | | | | |
|---|---|---|---|---|---|
| | Day 0 | Day 1 | Day 3 | Day 5 | Day 7 |
| G1 blank control group | 0.0 | 0.0 | 0.0 | 0.0 | 0.0 |
| G2 negative control group | 0.0 | 0.0 | 6.1±0.59 | 8.3±0.65 | 9.6±0.29 |
| G3. Tofacitinib 30mg/kg | 0.0 | 0.0 | 4.6±0.50 | 4.9±0.42*** | 2.8±0.43*** |
| G4. Crystalline form A 0.5mg/kg | 0.0 | 0.0 | 6.6±0.69 | 5.9±0.79** | 6.1±0.56*** |
| G5. Crystalline form A 1.5mg/kg | 0.0 | 0.0 | 6.1±0.56 | 7.0±0.53 | 4.9±0.39*** |
| G6. Crystalline form A 5mg/kg | 0.0 | 0.0 | 4.9±0.48 | 4.7±0.58*** | 3.6±0.44*** |

| | | | | | |
|---|---|---|---|---|---|
| Note: * P<0.05, * * P<0.01, * * * P<0.001 are all compared with the G2 negative control group | | | | | |

Result analysis: On the 7th day of treatment, the G4 group treated with crystalline form A (0.5 mg/kg, twice a day) showed a significant improvement in psoriasis symptoms compared to the G2 negative control group with an average PASI score of 6.1, and there was a statistically significant difference (P<0.05).

On the 7th day of treatment, the G5 group treated with crystalline form A (1.5 mg/kg, twice a day) and the G6 group treated with crystalline form A (5 mg/kg, twice a day) had an average double layer skin thickness of 1.15mm and 1.03mm on the back, respectively. The average PASI score was 4.9 and 3.6 points, respectively. Compared with the G2 negative control group, the G6 group treated with crystalline form A (5 mg/kg, twice a day) had a significant inhibitory effect on inflammatory thickening of the back skin and significantly improved the symptoms of psoriasis. The inhibition rates of back skin thickening were 56.4% and 71.8%, respectively, There were significant differences in statistics (P<0.001).

From the above content, it can be seen that the compound (R)-2-(1-(2-(1-hydroxyethyl)imidazolo[4,5-d]pyrrolo[2,3-b]pyridine-1(6H)-yl)piperidin-4-yl) acetonitrile free base provided by the present invention has a new crystal form, good stability, high solubility, and has inhibitory effects on inflammatory thickening of the back skin and auricle in mice, which can effectively improve the incidence of specific dermatitis and psoriasis; The preparation method is easy to operate and has good reproducibility, which can meet the needs of large-scale industrial production.

The various embodiments in this manual are described in relevant ways, and the same and similar parts between each embodiment can be referred to each other. Each embodiment focuses on the differences from other embodiments.

The above is only a preferred embodiment of the present invention and is not intended to limit it. Any modifications, equivalent substitutions, improvements, etc. made within the spirit and principles of the present invention shall be included within the scope of protection of the present invention.

## Claims

1. A crystalline form A of the compound of formula (I), 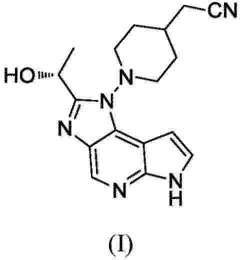 wherein the X-ray powder diffraction pattern comprises characteristic peaks at 2θ angles of 8.6°±0.2°, 9.8°±0.2°, 10.7°±0.2°, 11.2°±0.2°, 12.3°±0.2°, 12.9°±0.2°, 16.0°±0.2°, and 19.0°±0.2°.

2. The crystalline form A of claim 1, wherein the X-ray powder diffraction pattern comprises characteristic peaks at 2θ angles of 8.6°±0.2°, 9.8°±0.2°, 10.7°±0.2°, 11.2°±0.2°, 12.3°±0.2°, 12.9°±0.2°, 16.0°±0.2°, 17.0°±0.2°, 17.4°±0.2°, 17.8°±0.2°, 19.0°±0.2°, 19.9°±0.2°, and 21.2°±0.2°.

3. The crystalline form A of claim 1 or 2, wherein the X-ray powder diffraction pattern is substantially as shown in Table 1.

4. The crystalline form A of any one of claims 1-3, wherein the X-ray powder diffraction pattern is substantially as shown in Figure 2.

5. A crystalline form Q of the compound of formula (I), 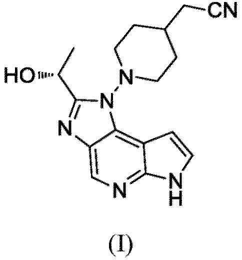 wherein the X-ray powder diffraction pattern comprises characteristic peaks at 2θ angles of 8.5°±0.2°, 14.5°±0.2°, 15.0°±0.2°, 16.8°±0.2°, 19.4°±0.2°, 23.6°±0.2°, 24.8°±0.2°, 26.3°±0.2°, and 27.4°±0.2°.

6. The crystalline form Q of claim 5, wherein the X-ray powder diffraction pattern comprises characteristic peaks at 2θ angles of 8.5°±0.2°, 14.5°±0.2°, 15.0°±0.2°, 15.8°±0.2°, 16.8°±0.2°, 18.8°±0.2°, 19.4°±0.2°, 20.1°±0.2 °, 23.6°±0.2°, 24.8°±0.2°, 26.3°±0.2°, and 27.4°±0.2°.

7. The crystalline form Q of claim 5 or 6, wherein the X-ray powder diffraction pattern is substantially as shown in Figure 13.

8. A pharmaceutical composition, wherein the pharmaceutical composition comprises an effective therapeutic amount of the crystalline form A of any one of claims 1-4 or the crystalline form Q of any one of claims 5-7 or a mixture thereof, and pharmaceutically acceptable carriers, diluents or excipients.

9. A veterinary pharmaceutical composition, wherein the pharmaceutical composition comprises an effective therapeutic amount of the crystalline form A of any one of claims 1-4 or the crystalline form Q of any one of claims 5-7 or a mixture thereof, and pharmaceutically acceptable carriers, diluents or excipients.

10. A method for preparing crystalline form A of any one of claims 1-4, wherein the method comprises: dissolving the compound of formula (I) in ethanol until it is clear, and then concentrating; stirring the obtained concentrate, a solid precipitated; filtered and dried to obtain the crystalline form A.

11. Use of the crystalline form A of any one of claims 1-4 or the crystalline form Q of any one of claims 5-7 or the pharmaceutical composition of claim 8 or veterinary pharmaceutical composition of claim 9 in the manufacture of a medicament.

12. Use of claim 11, wherein the medicament is used to treat, prevent or delay allergic reactions, allergic dermatitis, atopic dermatitis, eczema, itching or psoriasis.

13. Use of claim 12, wherein the medicament is used to treat, prevent, or delay allergic reactions, allergic dermatitis, atopic dermatitis, eczema, pruritus, or psoriasis in mammals.

14. Use of claim 13, wherein the mammal includes a companion animal.

15. Use of claim 13 or 14, wherein the mammal includes livestock.

16. The use of any one of claims 11-15, wherein the medicament is used to treat diseases mediated by JAK kinase.

17. A method for treating and/or preventing diseases, wherein the method comprises administering a therapeutically effective amount of the crystalline form of any one of claims 1-7 or the pharmaceutical composition of claim 8 or the veterinary pharmaceutical composition of claim 9 to a mammal in need.

18. The method of claim 17, wherein the therapeutically effective amount is from 0.01 mg/kg body weight/day to 100 mg/kg body weight/day.

19. The method of claim 17 or 18, wherein the therapeutically effective amount is from 0.1 mg/kg body weight/day to 30 mg/kg body weight/day.

20. The method of any one of claims 17-19, wherein the disease is mediated by JAK kinase.

21. The method of any one of claims 17-20, wherein the mammal includes a companion animal.

22. The method of any one of claims 17-21, wherein the mammal includes a domestic animal.

23. The method of any one of claims 17-22, wherein the administering is performed orally, parenterally or topically.
